Europäisches Patentamt

European Patent Office

Office européen des brevets

(11) Publication number: **0 416 739 A1**

# EUROPEAN PATENT APPLICATION

(21) Application number: 90308499.4

(22) Date of filing: 01.08.90

(51) Int. Cl.⁵: **A61K 31/52**, A61K 47/10, A61K 47/20

The title of the invention has been amended (Guidelines for Examination in the EPO, A-III, 7.3).

(30) Priority: 05.08.89 GB 8917959

(43) Date of publication of application:
13.03.91 Bulletin 91/11

(84) Designated Contracting States:
AT BE CH DE DK ES FR GB GR IT LI LU NL SE

(71) Applicant: **Beecham Group p.l.c.**
**SB House Great West Road**
**Brentford Middlesex TW8 9BD(GB)**

(72) Inventor: **Griffiths, Hazel-Anne**
**SmithKline Beecham Pharmaceuticals,**
**Clarendon Road**
**Worthing, West Sussex BN14 8QH(GB)**
Inventor: **Sanderson, Francis Dominic**
**SmithKline Beecham Pharmaceuticals,**
**Clarendon Road**
**Worthing, West Sussex BN14 8QH(GB)**

(74) Representative: **Jones, Pauline et al**
**SmithKline Beecham, Corporate Patents,**
**Great Burgh, Yew Tree Bottom Road**
**Epsom, Surrey KT18 5XQ(GB)**

(54) Pharmaceutical compositions containing penciclovir.

(57) An oil-in-water or an aqueous topical pharmaceutical formulation for the treatment of virus infections of the skin or mucosa, comprising at least 30% propylene glycol, 0.1 to 10% decylmethylsulphoxide and solubilised penciclovir.

EP 0 416 739 A1

# PHARMACEUTICAL FORMULATION

This invention relates to a topical pharmaceutical formulation suitable for use in the treatment of virus infections of the skin and mucosa.

EP-A-141927 (Beecham Group p.l.c.) discloses the compound 9-(4-hydroxy-3-hydroxymethylbut-1-yl)-guanine, known as BRL 39123 or penciclovir, its salts and esters thereof and its use in the treatment of herpesvirus infections. Topical administration is disclosed as a suitable route. Hereinafter, references to penciclovir include salts and esters thereof.

It is important with a topical formulation of an antiherpesvirus drug that the quantity of drug released from the formulation is sufficient to exert a significant antiviral effect and that the drug rapidly reaches its site of action within the skin.

Rapid penetration is important, since in most cases, major virus induced epidermal pathology occurs within the first 24 hours post infection. Once the infection is established and the stratum corneum has been eroded, the rapid ingress of host resistant factors could make chemotherapy of questionable value.

Propylene glycol has been used in a topical pharmaceutical formulation of acyclovir, used for treating viral infections (EP-A-44543).

Use of decylmethylsulphoxide (DecMSO) has been described as a penetration enhancer in topical formulations of topically active drugs, however when it has been used in combination with a propylene glycol formulation, at concentrations of up to 15%, DecMSO produced only a marginal increase in penetration of the drug (Goodman and Barry, Journal of Investigative Dermatology Vol. 91, No.4, October 1988).

A formulation containing DecMSO and propylene glycol has now been discovered which shows surprisingly high levels of penetration of penciclovir, within 24-29 hours of application. Depending on the concentration of DecMSO used, an appreciable proportion of the compound will penetrate the skin within 8 hours of application of the formulation.

Accordingly, the present invention provides an oil-in-water or an aqueous topical pharmaceutical formulation for the treatment of virus infections of the skin or mucosa, comprising at least 30% propylene glycol, 0.1% to 10.0% decylmethylsulphoxide and solubilised penciclovir.

Favourably, the formulation will contain 0.1 to 3.0% decylmethylsulphoxide.

Such a topical formulation may contain 0.075% to 10% w/w penciclovir and from 30% to 60% w/w of propylene glycol and from 15% w/w water (up to 50% when there is an oil phase).

In one aspect the formulation comprises from 1% to 10% w/w penciclovir, from 30% to 50% w/w of propylene glycol, 0.5 to 2.5% DecMSO, from 20% w/w water (up to 40%, when there is an oil phase), more particularly comprising from 2% to 5% w/w penciclovir, from 35% to 45% w/w of propylene glycol, 1-2% DecMSO, from 25% to 40% w/w water together with an oil phase. In a preferred aspect the formulation comprises from 1% to 10% w/w penciclovir, from 30% to 50% w/w of propylene glycol, 1.0 to 10.0% DecMSO, from 20% w/w water (up to 40%, when there is an oil phase), whilst the most preferred formulation comprises from 2% to 5% w/w penciclovir, from 35% to 45% w/w of propylene glycol, 1-3% DecMSO, from 25% to 40% w/w water together with an oil phase. The formulation should preferably contain about 40% w/w of propylene glycol.

The amount of penciclovir present in the formulation should be at least sufficient to maintain an antivirally effective concentration at the site of infection between applications without showing signs of toxicity. The optimum concentration of penciclovir will depend on its solubility in the vehicle. Penciclovir may be included in the formulation at a level exceeding its solubility in order to provide a reservoir and to maintain the antiviral agent at a saturated concentration within the vehicle. A particularly suitable amount in the above preferred formulation is 2% w/w.

The water used in the formulation is preferably purified water, purified that is by distillation by means of ion exchange, or other appropriate method.

The oil phase of the emulsions of this invention may be constituted from known ingredients in a known manner. While the phase may comprise merely an emulsifier (otherwise known as a emulgent), it is desirably comprised of a mixture of at least one emulsifier with one or more excipients including oils, fats and/or waxes, together with optional film formers and stabilisers as well as thickening and bodying agents. DecMSO itself has surface active properties and may be used as the sole emulsifier or in combination with another emulsifier. Preferably, as explained in more detail below, an additional hydrophilic emulsifier is included together with a lipophilic emulsifier which acts as a stabiliser. It is also preferred to include both an oil and a fat. Together, the emulsifier(s) make up the so-called emulsifying wax, and the wax together with the oil and/or fat make up the so called emulsifying ointment base which forms the oil dispersed phase of the emulsions.

Oil-in-water topical formulations may be formulated in a number of ways, all of which depend primarily on the alignment of the emulgent or emulsifying agent and emulsion stabiliser at the oil/water interface, with the non-polar or lipophilic groups soluble in the oil phase and the polar or hydrophilic or lipophilic groups in the aqueous or continuous phase. Thus the more polar hydrophilic emulgents result in oil-in-water emulsions. This principle has been systemised in the idea of a 'hydrophilic-lipophilic balance' (H.L.B.) Griffen, W. C. J. Soc. Cosmet. Chem ., 1954, 5 , 249 and the various emulgents have been allocated H.L.B. numbers from which their behaviour with constituents of he aqueous and oil phases (to which are applied theoretical required H.L.B. figures) may be predicted.

Emulgents and emulsion stabilisers suitable for use in the formulation of the present invention include polyoxyethylene sorbitan monostearate (polysorbate 60), sorbitan monostearate, sorbitan monooleate, cetostearyl alcohol, myristyl alcohol, glyceryl monostearate and sodium lauryl sulphate.

The choice of suitable oils or fats for the formulation is based on achieving the desired cosmetic properties. Thus the cream should preferably be non-greasy, non-staining and washable product with suitable consistency to avoid leakage from tubes or other containers. Lipophilic substances with relatively high melting points, such as beeswax, partial glycerides of capric and caprylic acids, or silicone oil, white soft paraffin and/or liquid paraffin or other mineral or vegetable oils are suitable. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a mixed ester of 2-ethyl hexanoic acid with a blend of cetyl or stearyl alcohols known as Crodamol CAP may also be used.

As well as creams, the aqueous/oil-in-water formulation may be a lotion, skin paint, gel, spray, aerosol, liniment or gel stick, which are formulated as known in the art, for example as described in standard text books of pharmaceutics and cosmetics, such as Harry's Cosmeticology published by Leonard Hill Books, the British Pharmacopoeia, twenty-first revision (USP XXI) (1984), distributed by Mack Publishing Company.

The product may or may not be sterile, with adequate preservative capacity for single or multi-dose purpose

In this description, the following terms are employed:

Aerosol - Pharmaceutical aerosols are products that are packaged under pressure and contain therapeutically active ingredients that are released upon activation of an appropriate valve system. The term 'aerosol' has been used to refer to the fine mist of spray that is emitted from a pressurized container containing an active ingredient and a propellant. However, the term has been broadly applied to include all self-contained pressurized products, some of which deliver foams or semisolid fluids. Accordingly, unless indicated otherwise, a reference herein to an aerosol formulation of the present invention should be understood to comprising a pharmaceutically acceptable carrier which includes a propellant, said compositions being adapted for use in a pressurized container that dispenses the composition as a spray, foam or semisolid liquid.

An aerosol generally comprises a container, a propellant, a concentrate containing the active ingredient, a valve (which may be a metered valve), and an actuator. The nature cf these components determines characteristics such as delivery rate, foam density, and fluid viscosity. Aerosols may be two-phase (gas and liquid) or three-phase (gas, liquid, and solid or liquid formulations. A two-phase formulation consists of a solution of active ingredients in liquidified propellant and the vaporized propellant. The solvent may be the propellant or a mixture of the propellant and co-solvents such as alcohol and polyethylene glycols which are often used to enhance the solubility of the active ingredients. Three-phase formulations consist of a suspension or emulsion of the active ingredient(s) in addition to the vaporized propellants. A suspension consists of the active ingredient(s) dispersed in the propellant system with the aid of suitable excipients such as wetting agents and/or solid carriers such as talc or colloidal silicas. A foam formulation is generally an emulsion containing one or more active ingredients, surfactants, aqueous or nonaqueous liquids, and the propellants. If the propellant is in the internal (discontinuous) phase (i.e., of the oil-in-water type), a stable foam is discharged, and if the propellant is in the external (continuous) phase (i.e., of the water-in-oil type), a spray or a quick-breaking foam is discharged. (See The United States Pharmacopoeia , XXI ('USP') at 1334].

Gels - Gels are semisolid systems consisting either of suspensions made up of small inorganic particles or of large organic molecules interpenetrated by a liquid. Where the gel consists of a network of small discrete particles, the gel is classified as a two-phase system. In a two-phase gel, if the particle size of the dispersed phase is relatively large, the gel is sometimes referred to as a magma. Both gels and magmas

3

EP 0 416 739 A1

may be thixotropic, forming semisolids on standing and becoming liquid on agitation.

Single-phase gels consist of organic macromolecules uniformly distributed throughout a liquid so that no apparent boundaries exist between the dispersed macromolecules and the liquid. Single-phase gels may be made from synthetic macromolecules (e.g. Carbomer ) * or from natural gums (e.g. Tragacanth ). The latter preparations are also called mucilages. Although single-phase gels are commonly aqueous, alcohols and oils may also be used as the continuous phase. For example, mineral oil can be combined with a polyethylene resin to form a gel which may be used as an oleaginous ointment base [see USP , supra at 1336].

Lotion - Preferred lotions include fluid or thixotropic emulsions intended for external application to the body. These lotions are emulsions of the oil-in-water type stabilized by a surface-active agent. They may separate or stratify on long standing, and should be well shaken before each use. Adequate preservation against microbial contamination is required [see USP , supra at 1337].

Gel stick - The definition of gel sticks set forth in Harry's Cosmeticology , 6th Edition, at 740, is hereby incorporated herein by reference.

Liniment - Liniments are solutions or mixtures of various substances in oil, alcoholic solutions of soap, or emulsions, as in the present invention. They are intended for external application and are usually applied with friction and rubbing of skin, the oil or soap base providing for ease of application and massage.

Spray - As used in this description, spray formulations are aqueous solutions of various drugs which are applied topically from a container having a spray means (e.g., an atomizer or nebulizer).

The present invention further provides a method for the preparation of a topical pharmaceutical formulation, as hereinbefore defined, which comprises admixing the combination of penciclovir, propylene glycol, DecMSO and water, optionally with oil phase.

The manner of formulating an emulsion will of course vary according to the amount and nature of the constituents, but nevertheless follows known techniques in emulsion technology (see The Pharmaceutical Codex, London, The Pharmaceutical Press, 1979). In a preferred method penciclovir may be included in the emulsifying ointment prior to emulsification with the aqueous portion. Alternatively the penciclovir may be initially incorporated wholly in the aqueous portion where it may form a solution alone, or mixed solution/suspension, and then emulsified with the ointment base, or a part of the aqueous portion may be formulated as an emulsion, and the balance of the water, propylene glycol and penciclovir added to and dispersed into the emulsion. In using these procedures, it is preferable to heat the aqueous portion and the ointment base to about 40 to 80°C, preferably 50 to 70°C, prior to emulsification which may be achieved by vigorous agitation using for example a standard laboratory mixer. Finer dispersions of the oil phase may be obtained by homogenising or milling in a colloidal mill.

A topical formulation of the present invention may be used in the treatment or prevention of viral infections caused by herpesviruses such as herpes simplex types 1 and 2 and varicella-zoster virus.

The formulation should be applied in the infected area 2 to 6 times daily, preferably 2 or 3 times.

The following examples illustrate the invention.

Tables 1 and 2 show the effect of decylmethylsulphoxide concentration on the in vitro percutaneous penetration of penciclovir through human skin.

CREAMS CONTAINING PROPYLENE GLYCOL AND DecMSO

*Formulations that are capitalized in this discussion are classified as pharmacopoeial preparations (USP).

4

| Example 1 | |
|---|---|
| Oil phase | % w/w |
| Polawax NF | 18.0 |
| White soft paraffin | 10.0 |
| DecMSO | 2.0 |
| BRL 39123 (penciclovir) | 5.0 |
| Aqueous phase | |
| Sodium lauryl sulphate | 0.75 |
| Propylene glycol | 40.0 |
| Distilled water to | 100.0 |

| Example 2 | |
|---|---|
| Oil phase | % w/w |
| Cetostearyl alcohol | 6.72 |
| White soft paraffin | 13.78 |
| Glyceryl monostearate | 7.50 |
| DecMSO | 1.5 |
| BRL 39123 (penciclovir) | 2.0 |
| Aqueous phase | |
| Sodium lauryl sulphate | 0.75 |
| Propylene glycol | 40.0 |
| Distilled water to | 100.0 |

| Example 3 | |
|---|---|
| Oil phase | % w/w |
| Cetostearyl alcohol | 7.26 |
| Liquid paraffin | 19.55 |
| **Carbopol 940 | 1.0 |
| DecMSO | 1.0 |
| BRL 39123 (penciclovir) | 1.0 |
| Aqueous phase | |
| Propylene glycol | 40.0 |
| Distilled water to | 100.0 |

| Example 4 | |
|---|---|
| Oil phase | % w/w |
| Cetostearyl alcohol | 7.8 |
| White soft paraffin | 15.0 |
| Glyceryl monostearate | 4.0 |
| ***Span 80 | 1.55 |
| DecMSO | 1.0 |
| BRL 39123 (penciclovir) | 0.5 |
| Aqueous phase | |
| Propylene glycol | 40.0 |
| ****Tween 80 | 3.45 |
| Distilled water to | 100.0 |

** Carboxyvinyl polymer
*** Sorbitan mono-oleate
**** Polysorbate 80 [polyoxyethylene(20)sorbitan mono-oleate]

| Example 5 | |
|---|---|
| Oil phase | % w/w |
| Cetostearyl alcohol | 7.26 |
| Liquid paraffin | 19.55 |
| **Carbopol 940 | 1.0 |
| DecMSO | 2.5 |
| BRL 39123 (penciclovir) | 1.0 |
| Aqueous phase | |
| Propylene glycol | 40.0 |
| Distilled water to | 100.0 |

Method of Manufacture

Components of the oil phase were heated to 65-70°C, with constant stirring, until molten. The BRL 39123 (penciclovir) was added to the molten oil phase and homogenised for 5 minutes. The aqueous phase was heated to 65-70°C and stirred until complete solution was achieved. This was then added to the oil phase, kept at the same temperature and homogenised for 10 minutes. The cream was stirred while cooling and at 40-45°C, a vacuum of 0.9 bar was pulled. Stirring was continued until the cream reached a temperature of 25-30°C. Finally the cream was packed into suitable containers.

Effect of Decylmethylsulphoxide concentration on the 'in vitro' Percutaneous Penetration of Penciclovir

6

Table 1

| Concentration of DecMSO in 40% P.G. Cream Formulation % | Amount penetrated After 29 hours $\mu$g cm$^{-2}$ |
|---|---|
| 1.0 (no PG) | 0.69 |
| 1.0 | 15.03 |
| 0.8 | 9.86 |
| 0.6 | 10.27 |
| 0.4 | 6.08 |
| 0.2 | 3.85 |
| 0.1 | 2.31 |

Table 2

| Concentration of DecMSO in 40% P.G. Cream Formulation % | Amount penetrated $\mu$g cm$^{-2}$ | |
|---|---|---|
| | 7.75 hours | 23.75 hours |
| 0 | ND | 0.91 |
| 1.0 | ND | 6.91 |
| 2.5 | 1.70 | 27.64 |
| 10.0 | 5.77 | 38.79 |
| P.G. = polyethylene glycol. ND = not detectable | | |

## Claims

1. An oil-in-water or an aqueous topical pharmaceutical formulation for the treatment of virus infections of the skin or mucosa, comprising at least 30% propylene glycol, 0.1 to 10% decylmethylsulphoxide and solubilised penciclovir.

2. A pharmaceutical formulation according to claim 1 comprising 0.1 to 3.0% decylmethylsulphoxide.

3. A pharmaceutical formulation according to claim 1 or 2 containing from 0.075 to 10% penciclovir, 30 to 60% propylene glycol and at least 15% water.

4. A pharmaceutical formulation according to claim 3 comprising from 1 to 10% penciclovir, 30-50% propylene glycol, 1 to 10% decylmethylsulphoxide and at least 20% water.

5. A pharmaceutical formulation according to claim 4 comprising 2 to 5% penciclovir, 35 to 45% propylene glycol, 1 to 3% decylmethylsulphoxide and 25 to 40% water together with an oil phase.

6. A pharmaceutical formulation according to any one of claims 1 to 5, comprising 2% penciclovir.

7. A method for the preparation of a pharmaceutical formulation according to claim 1, which method comprises admixing the combination of penciclovir, propylene glycol, decylmethylsulphoxide and water, optionally with an oil phase.

8. A pharmaceutical formulation according to claim 1 for use as an active therapeutic substance.

9. A pharmaceutical formulation according to claim 1 for use in the treatment of viral infections.

10. Use of a pharmaceutical formulation according to claim 1 in the manufacture of a medicament for use in the treatment of viral infections.

Claims for the following Contracting States: ES, GR.

1. A method for the preparation of an oil-in-water or an aqueous topical pharmaceutical formulation for the treatment of virus infections of the skin or mucosa, comprising at least 30% propylene glycol, 0.1 to 10% decylmethylsulphoxide and solubilised penciclovir; which method comprises admixing the combination of penciclovir, propylene glycol, decylmethylsulphoxide and water, optionally with an oil phase.

2. A method according to claim 1 for the preparation of a pharmaceutical formulation comprising 0.1 to 3.0% decylmethylsulphoxide.

3. A method according to claim 1 or 2 for the preparation of a pharmaceutical formulation containing from 0.075 to 10% penciclovir, 30 to 60% propylene glycol and at least 15% water.

4. A method according to claim 3 for the preparation of a pharmaceutical formulation comprising from 1 to 10% penciclovir, 30-50% propylene glycol, 1 to 10% decylmethylsulphoxide and at least 20% water.

5. A method according to claim 4 for the preparation of a pharmaceutical formulation comprising 2 to 5% penciclovir, 35 to 45% propylene glycol, 1 to 3% decylmethylsulphoxide and 25 to 40% water together with an oil phase.

6. A method according to any one of claim 1 to 5, for the preparation of a pharmaceutical formulation comprising 2% penciclovir.

7. Use of a pharmaceutical formulation as defined in claim 1, in the manufacture of a medicament for use in the treatment of viral infections.

European
Patent Office

**EUROPEAN SEARCH
REPORT**

Application Number

**EP 90 30 8499**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| D,A | EP-A-0 044 543   (WELLCOME)<br>* Claims 1,6,11-12 * | 1,7-10 | A 61 K 31/52<br>A 61 K 47/10<br>A 61 K 47/20 |
| D,A | EP-A-0 141 927   (BEECHAM)<br>* Claims 1,12; page 16, lines 20-26 * | 1,7-10 | |
| A | EP-A-0 152 316   (MERCK)<br>* Claims 1,6,11; page 13, lines 1-8 * | 1,7-10 | |
| A | EP-A-0 146 516   (ASTRA)<br>* Claims 1,15-16,19-20; page 25, lines 1-11 * | 1,7-10 | |
| A | US-A-3 839 566   (F.S. KILMER)<br>* Claims; column 2, lines 29-40 * | 1,7-10 | |

|  | TECHNICAL FIELDS SEARCHED (Int. Cl.5) |
|---|---|
|  | A 61 K |

The present search report has been drawn up for all claims

| Place of search | Date of completion of search | Examiner |
|---|---|---|
| The Hague | 08 November 90 | SCARPONI U. |